(19) Europäisches Patentamt
European Patent Office
Office européen des brevets

(11) **EP 0 541 950 B1**

(12) **EUROPEAN PATENT SPECIFICATION**

(45) Date of publication and mention
of the grant of the patent:
**10.03.2004 Bulletin 2004/11**

(51) Int Cl.⁷: **A61B 17/064**, A61B 17/068

(21) Application number: **92116976.9**

(22) Date of filing: **05.10.1992**

(54) **Stapling device comprising malleable, bioabsorbable, plastic staples**

Klammergerät beinhaltend aus dehnbarem, bioabsorbierbarem Kunststoff hergestellte Klammern

Dispositif comprenant des agrafes en matière plastique malléable et bioabsorbable

(84) Designated Contracting States:
**AT BE CH DE DK ES FR GB GR IE IT LI LU NL PT SE**

(30) Priority: **30.10.1991 US 785295**
**30.06.1992 US 906455**

(43) Date of publication of application:
**19.05.1993 Bulletin 1993/20**

(73) Proprietor: **Sherwood Services AG**
**8201 Schaffhausen (CH)**

(72) Inventors:
• **Esposito, Felix F.**
**Stratford, Connecticut 06497 (US)**
• **Logan, Joseph N.**
**Trunbull, Connecticut 06611 (US)**

• **Conners, John A.**
**Fairfield, Connecticut 06430 (US)**
• **Dwyer, James W.**
**Brookfield, Connecticut 06805 (US)**
• **Crainich, Laurence**
**Charlestown, New Hampshire 03603 (US)**

(74) Representative:
**Wächtershäuser, Günter, Prof. Dr.**
**Wächtershäuser & Hartz**
**Weinstrasse 8**
**80333 München (DE)**

(56) References cited:
WO-A-82/00582          WO-A-83/00614
FR-A- 325 704          GB-A- 1 339 394
GB-A- 2 051 287          GB-A- 2 075 144
GB-A- 2 233 903

## EP 0 541 950 B1

**Description**

The present invention relates to a stapling device for applying surgical staples, used to deform such staples to secure adjacent layers of tissue together. More specifically, this invention is defined in claim 1 and relates to a stapling device for applying malleable, bioabsorbable, polymeric staples for suturing body organs and tissue, and including a precision-formed anvil for deforming the staples into that suturing configuration.

A surgical stapler apparatus is known from WO 83/00614, which is used for stapling staples made of metal wire.

Gastrointestinal anastomosis-type devices drive and bend the staples aligned in a row one after the other in rapid sequence. Transverse anastomosis-type devices, drive and bend all staples in a row simultaneously.

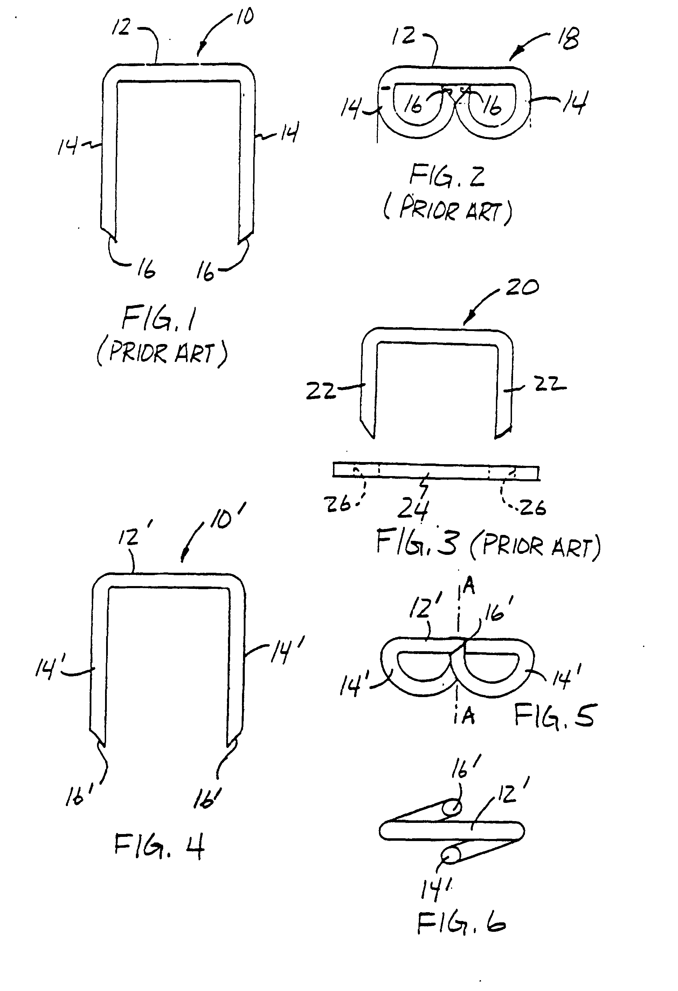

One type of conventional staple 10, shown in Figure 1, used with both gastrointestinal anastomosis and transverse anastomosis-type surgical stapling devices is made of a metal, like stainless steel or titanium, that is substantially inert in the body. The undeformed staple 10, or staple blank, is generally U-shaped and includes a back span 12 and two legs 14 depending perpendicularly from the back span in parallel to one another. Each leg 14 has a sharp chiseled end point 16 for cleanly piercing body organs or tissue. The metal staple blank is bent by having the legs engage and follow a conventional anvil to form a B-shaped closed staple 18 as shown in Figure 2.

The anvil used to bend metal surgical staples is also formed of a hardened metal and includes a staple-bending face having a pair of coined or punched pockets located to oppose each staple in the magazine of the stapling device. The pockets are ordinarily elongated arcuate depressions, co-linearly arranged in parallel to the back span of a corresponding staple held in the magazine. Thus the anvil closely resembles the anvil of a conventional paper stapler.

When the staples 10 are driven from the magazine toward the anvil, the staple legs 14 each engage one pocket so that both legs are bent toward each other initially and thereafter upwardly toward the back span 12. Thus, as shown in Figure 2, the end points 16 may come to rest against the underside of the back span 12.

Although metal staples inserted in the manner described above provide an effective and relatively simple means of suturing, one significant disadvantage is that they remain in the patient's body permanently. While generally not injurious to the body they may nevertheless interfere with post-operative X-ray or other diagnostic imaging of the patient.

This disadvantage can be overcome by using bioabsorbable polymeric staples that are degradable in the body after a short period of time. However, conventional polymeric staples are not malleable and thus cannot be easily bent into the B-shaped configuration shown in Figure 2, to complete a suture. Therefore, as shown in Figure 3, such conventional bioabsorbable staples instead are made in two parts, namely U-shaped polymeric staple body 20, the legs 22 of which are joined by a polymeric bar-like closure 24. The closure has two end point-receiving holes 26 that fit over the end points of the staple body 20 after they have pierced the tissue to be sutured. The staple body 20 and closure 24 are then forced toward each other to complete the suture.

While this two-part staple will dissolve in the body and, therefore, does not interfere with post-operative procedures, it has the drawback of requiring a part in addition to the basic staple blank and thus requires a more complicated mechanical stapling device for properly aligning the two parts and driving them together.

More recently, a breakthrough has been made in the bioabsorbable staple field. Specifically, bioabsorbable or partially bioabsorbable surgical staples have been developed using polymeric materials. These staples retain all of the beneficial attributes of known bioabsorbable staples, but in addition are malleable or plastically deformable like metal staples. That is, these staples may be bent into complex shapes that are then retained. Therefore, they may be made of a single piece, not requiring independent staple body and closure parts.

Nevertheless, it has been found that if these new bioabsorbable staples are bent in the same way as are conventional metal staples, as shown in Figure 2, so that the chiseled end points of the staple legs hit the back span, the points may crush or break.

Therefore, further improvement in surgical staples and devices for inserting them, taking advantage of the attributes of the new polymeric materials described above, are desirable.

Accordingly, it is a principle object of the present invention to enhance the benefits obtained by using malleable, bioabsorbable, polymeric staples in surgical stapling techniques.

It is a further object of the present invention to provide a stapling device comprising a malleable, bioabsorbable, polymeric staple deformed into a precise shape that securely joins tissue sections together with minimal tissue injury and damage to the staple itself.

It is another object of the present invention to provide a stapling device comprising a malleable bioabsorbable, polymeric staple having a cross-sectional shape that enhances tissue or clinching strength once deformed.

It is another object of the present invention to provide a stapling device comprising a high precision anvil for surgical stapling devices that will precisely and uniformly deform malleable, bioabsorbable, polymeric staples, as well as other staples, into a desired configuration.

It is yet another object to provide a method for deforming the malleable, bioabsorbable, polymeric staple into the desired shape.

It is still another object of the present invention to provide a stapling device comprising a unique anvil that takes advantage of the beneficial properties of malleable, bioabsorbable, polymeric staples of the type described above.

These and other objects are achieved by the stapling device according to claim 1 including a bioabsorbable, polymeric surgical staple which comprises a back span, and first and second legs extending generally in the same direction from opposite ends of the back span, with the first and second legs having first and second end points, respectively. The first and second legs are deformed inwardly toward each other and upwardly toward the underside of the back span such that the end points of the respective legs extends past opposite sides of the back span.

The stapling device is used in a method of deforming a malleable, bioabsorbable, polymeric staple wherein in an initial undeformed configuration the staple has a back span and first and second legs each having an end point and each extending in the same direction from opposite ends of the back span substantially perpendicularly thereto. The method includes the steps of initially deforming the first and second legs inwardly toward each other, and thereafter deforming the first and second legs upwardly such that the first and second end points extend past the back span of the staple on opposite sides thereof.

The polymeric surgical staple also preferably has a noncircular oval or rectangular cross-sectional shape that enhances its ability to retain its deformed, tissue-joining configuration, as will be described in detail below.

Yet another aspect is a surgical stapling device anvil for forming a staple having, in an undeformed state, a back span and first and second legs extending in the same direction from opposite ends of the back span substantially perpendicularly thereto. The anvil comprises a supporting body having a longitudinal axis and including a staple-receiving face, that may confront the end points of the legs of the staple. First and second pocket-like depressions are formed in the supporting body and each begins with an entry end located at the face, continues to a depressed portion within the body below the face, and terminates in an exit end at the face. The first and second pocket-like depressions extend in non-collinear relation with the entry end of each located substantially on the longitudinal axis and the exit end of each located on a side of the axis opposite the side on which the exit end of the other depression is located. The entry ends of the first and second depressions are spaced by a distance substantially equal to the distance between the depending legs of a staple blank. Accordingly, a staple such as described above driven toward this anvil will be deformed by first bending the staple legs toward each other and thereafter upwardly toward the back span. However, the end points of the legs will be steered toward opposite sides of the back span past the back span.

It will be appreciated, of course, that the surgical stapling device anvil configured in accordance with the present invention may be used with surgical staples of any material. However, because it is specifically designed for use with malleable, bioabsorbable, polymeric staples that are non-metallic, it may be made of plastic materials that are less expensive and in which the high precision pocket-like depressions may be more easily formed than known hardened metal anvils.

These and other objects, aspects, features, and advantages of the present invention will become apparent from the following detailed description of the preferred embodiments taken in conjunction with the drawings.

DRAWINGS

Figure 1 a front elevational view of a conventional metal staple bank made, for example, of stainless steel or titanium;

Figure 2 is a front elevational view of a conventional staple in a deformed configuration;

Figure 3 is a front elevational view of a conventional two-piece bioabsorbable polymeric staple;

Figure 4 is a front elevational view of a malleable, bioabsorbable, polymeric staple, which is not yet deformed, in accordance with the present invention;

Figure 5 is a front elevational view of a malleable, bioabsorbable, polymeric staple in accordance with the present invention deformed to a shape for suturing adjacent tissue sections together;

Figure 6 is a top plan view of the staple shown in Figure 5 in its deformed state;

Figure 6A is a detailed cross-sectional view of one particular embodiment of the staple shown in Figure 5;

Figure 6B is a detailed cross-sectional view of another embodiment of the staple shown in Figure 5;

Figure 7 is a schematic perspective view of a surgical stapling device anvil which does not form part of the present invention and is shown only or reference purposes.

Figure 8 is a schematic top plan view of the surgical stapling device anvil shown in Figure 7;

Figure 9 is a vertical cross-sectional view of the surgical stapling device anvil shown in Figures 7 and 8, and taken on plane 9-9 in Figure 8.

Figure 10 is a schematic perspective view of a surgical stapling device anvil formed in accordance with the invention;

Figure 11 is a schematic top view of the anvil shown in Figure 10;

Figure 12 is a vertical cross-sectional view of the anvil shown in Figure 11 taken along plane 12-12;

Figure 13 is a vertical cross-sectional view of the anvil shown in Figure 11 taken along plane 13-13;

Figure 14 a vertical is cross-sectional view of the anvil shown in Figure 11 taken along plane 14-14;

Figure 15 is a vertical cross-sectional view similar to Figure 14, showing a staple point being received and steered in an anvil pocket toward a desired position;

Figure 16 is a vertical cross-sectional view similar to Figure 15, showing the staple point properly aligned in the anvil to be steered to the desired position;

Figure 17 is a top plan view of the anvil of Figures 10 through 14 and of a staple fully deformed thereby;

As noted above, surgical staples for use with a stapling device in accordance with the present invention are made of a polymeric or plastic material, some of the material being disclosed in the prior art. Because they are made of this material, these staples are plastically deformable or malleable as well as bioabsorbable. The present invention takes advantage of these unique properties to provide a stapling device including a surgical staple having an improved deformed configuration, obtainable by a method of deforming the staple to that configuration on a surgical stapling device anvil, the use of which results in that configuration. Of course other bioabsorbable or partially bioabsorbable malleable polymeric staples later developed may be adapted to the present invention.

More particularly, in its undeformed state shown in Figure 4, the surgical staple or staple blank 10' is generally U-shaped as are conventional staples shown in Figure 1. Thus the improved staple 10' also includes a back span 12', two legs 14', and an end point 16' formed at the extreme of each leg 14'. The end points are sharply chiseled to cleanly pierce the body organs or tissue to be sutured. However, while the polymeric staple is malleable, the end points may be brittle and can break or crush if pressed against a hard surface.

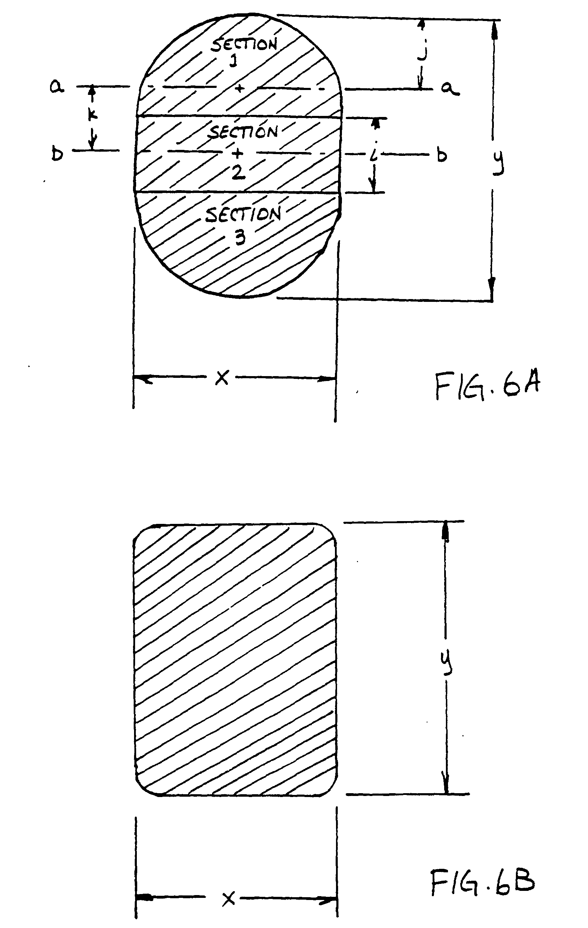

Figures 5 and 6 show the plastic staple 10' in its deformed state. As can be seen there, the legs 14' are bent from their configuration perpendicular to the back span 12' into an arcuate shape with the end points 16' extending toward opposite sides of the back span 12'. Thus the brittle end points 16' do not encounter the underside of the back span 12' during deformation and breaking or crushing of them is mitigated. Preferably, one end point 16' is guided toward one side of the back span and the other end point is guided toward the other side of the back span to further prevent the end points from engaging each other. The end points may desirably be closely adjacent opposite sides of the back span and may extend beyond or past the backspan. The end points can also be bent so that each extends in an opposite direction across an axial plane A-A perpendicular to the back span 12' of the staple.

While Figure 6 described above and Figure 17 to be described below illustrate the surgical staple of the present invention as having a generally circular cross-section, it is preferred that that cross-section be noncircular, for example, oval or rectangular, at least in the regions where the staple is to be bent.

More particularly, it is known that the flexural rigidity of a beam may be defined as E x I where E is the modules of elasticity of the beam material and I is the moment of inertia. I is determined by the cross-sectional shape of the beam. Therefore, beam stiffness can be controlled by appropriately determining the cross-sectional beam shape.

Using these principles, in one form the polymerical staple of the present invention has an oval cross sectional shape equal in cross-sectional area to a metal staple having a conventional circular cross-section, resulting in a 30 percent increase in beam stiffness without a resulting increase in the size of the tissue puncture area.

In greater detail, surgical polymeric staples that would otherwise be circular might arbitrarily have a diameter equal to about 0.0457 cm (0.018 inch) or a cross-sectional area A given as follows:

$$A = \pi \, \frac{d^2}{4} = 2.545 \times 10^{-4} \text{ inch}^2. \tag{1}$$

The moment of inertia I of such a staple is given by:

$$I = \pi \, (\frac{d^4}{64})$$

$$= 5.15 \times 10^{-9} \tag{2}$$

However, in accordance with one preferred embodiment of the present invention the cross-sectional shape is generally an oval as shown in Figure 6A. The cross-sectional area is equal to the sum of the areas of sections 1, 2 and 3, or:

$$A_{oval} = \pi \, (\frac{x^2}{4}) + x \, (y-x) \tag{3}$$

Setting the cross-sectional area of the oval staple to be equal to the cross-sectional area of the round staple for the reasons stated above, and empirically setting x to be 0.0381 cm (0.015 inch), then y = 0.0513 (0.0202 inch).
The moment of inertia $I_b$ of the staple of Figure 6A about axis b - b, that is assuming the staple will be bent up and down as shown in that figure, is given by the sum of the moments of inertia about that axis of sections 1, 2, and 3.
The moment of inertia of section 2 about axis b-b is given by the known formula:

$$I_{b\,(2)} = xi^3/12$$

$$= (0.015) \, (0.0052)^3/12$$

$$= 1.758 \times 10^{-10} \tag{4}$$

where i = y-x.
The moment of inertia of each of sections 1 and 3 about axis b - b is given by:

$$I_{b(1,3)} = I_a + A_{(1,3)}k^2 \tag{5}$$

where $I_a$ is given by the known formula:

$$I_a = 0.007x^4, \tag{6}$$

$A_{(1,3)}$ equals the cross-sectional area of section 1 or 3; and k equals the distance from axis a - a, which is the neutral axis or center of mass of sections 1 or 3, to axis b - b.
k is given by:

$$k = (\frac{y}{2}) - j \tag{7}$$

where j = 0.288x in accordance with the known formula.
Thus,

$$I_a = 0.007x^4$$

$$= 3.544 \times 10^{-10}$$

$$I_{b(1,3)} = I_a + A_{(1,3)}k^2$$
$$= (3.544 \times 10^{-10}) + (\pi x^2/8) \left[ \frac{0.0202}{2} \right] - (0.288)(.015)]^2$$
$$= (3.544 \times 10^{-10}) + (8.836 \times 10^{-5})(5.78 \times 10^{-3})^2$$
$$= 3.31 \times 10^{-9}$$

Accordingly, $I_{b(1,2,3)}$ for all three sections 1, 2, and 3 is

$$I_{b(1,2,3)} = I_{b(1)} + I_{b(2)} + I_{b(3)}$$

$$= 3.31 \times 10^{-9} + 1.758 \times 10^{-10} + 3.31 \times 10^{-9}$$

$$= 6.79 \times 10^{-9} \tag{8}$$

Comparing this value with that calculated using equation (2) for a staple having a round cross-section of equivalent area shows that the present invention achieves more than 30 percent greater beam stiffness than a staple having the circular cross-section of equivalent area.

Using the principles described above, polymeric surgical staples having other noncircular cross-sectional shapes than ovals are also possible. For example, another beneficial cross-sectional shape is the rectangle as shown in Figure 6B. In this embodiment, the moment of inertia I is given by

$$I = xy^3/12 \tag{9}$$

If x = 0.0381cm (0.015 inch) and y = 0.0513cm (0.0202 inch), which are the nominal dimensions of the oval staple described above, then

$$I = 1.03 \times 10^{-8}$$

This represents a 100 percent increase in beam stiffness above that provided by the comparable circular cross-section staple.

It is preferred that the aspect ratio of the minor dimension x to the major dimension y of the noncircular cross-section of the polymeric staple in accordance with the present invention is about 0.75. In the case described above, the aspect ratio is 0.74.

A precisely formed anvil is used to guide the polymeric staple components with the accuracy necessary to locate the end points 16' adjacent the back span 12' on opposite sides thereof. The end points should be guided sufficiently close to the back span so the stapled body organ cannot work its way off of the end points.

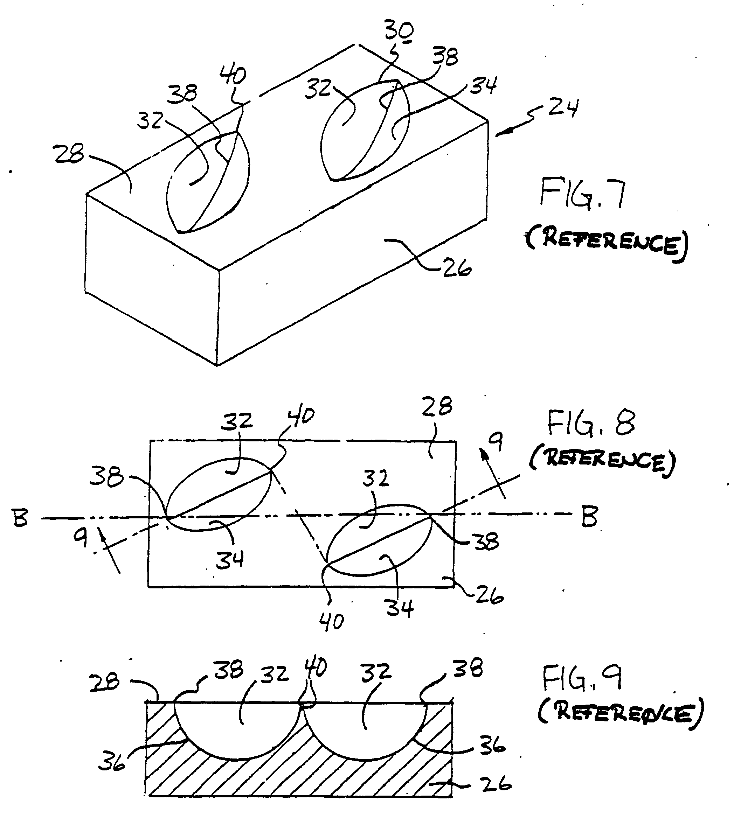

One such type of anvil 24 used to deform the polymeric staple is shown in Figures 7 through 9 for reference purposes. That anvil 24 has a supporting structure 26 having a staple-receiving face 28. The face and supporting structure can be either a one or multi-piece construction.

The face 28 includes two pockets 30 for receiving and guiding or steering the staple legs to the desired configuration.

It will be appreciated that while the anvil shown in Figures 7 through 9 includes but one pair of pockets 30, in the usual case an elongated row of pairs of such pockets would be formed in a similarly elongated support structure 26 so that a large number of surgical staples can be driven simultaneously or in rapid sequence. Each pocket is defined by opposing first and second walls 32 and 34 which slope downwardly and inwardly toward each other to meet and form an endpoint guiding path 36. The guiding paths 36 curve from respective entry ends 38 to exit ends 40 in the face 28. As also can be seen in Figures 7 and 8 the entry end 38 of each path 36 is located substantially on the longitudinal axis B-B of the anvil 24, but the paths also curve from the entry ends in opposite directions so that the respective exit ends 40 lie on opposite sides of the longitudinal axis.

The anvil is arranged in the surgical stapling device so that the longitudinal axis B-B is substantially parallel to the back span of a staple to be driven toward the anvil. Moreover, the entry ends 38 of the respective paths 36 are spaced so as to receive the respective end points 16' of the legs 14' of the staple driven toward the anvil. Accordingly, when the staple is so driven, the end points 16' each first encounter the entry end 38 of one guide path 36. As driving of the staple toward the anvil continues, the end points 16' are steered along the curved guide paths 36 ultimately to be pointed past opposite sides of the staple back span 12' when driving is completed. The anvil pockets 30 further are of suitable depth relative to the length of the staple legs to achieve this result and so that the staple end points are finally located on opposite sides of the axial plane A-A of the staple as shown in Figures 5 and 6.

Thus it can be seen that the surgical stapling device anvil will cause a malleable staple driven theretoward to assume as unique desired deformed configuration. Moreover, since this anvil is specifically designed to be used with malleable, bioabsorbable staples, which are made of polymeric material, it need not itself be made of a hardened material like metal. This factor is important because precisely shaped anvil pockets such as described above are difficult to form in hardened metal by other than very expensive machining techniques. Indeed coining or punching techniques for forming anvil pockets of conventional shape in known metal anvils are not suitable for forming the precisely shaped anvil pockets in accordance with the present invention. Thus plastics can be used to make the inventive anvil using precise yet inexpensive injection molding methods in the production process. Still further, plastics from which the anvil of the present invention may be made are themselves less expensive than metals used in conventional anvils. Therefore, the present invention provides significant advances over the prior art.

It has been found that polymeric materials like polycarbonate and liquid crystal polymer (LCP) may suitably be used for the inventive anvil.

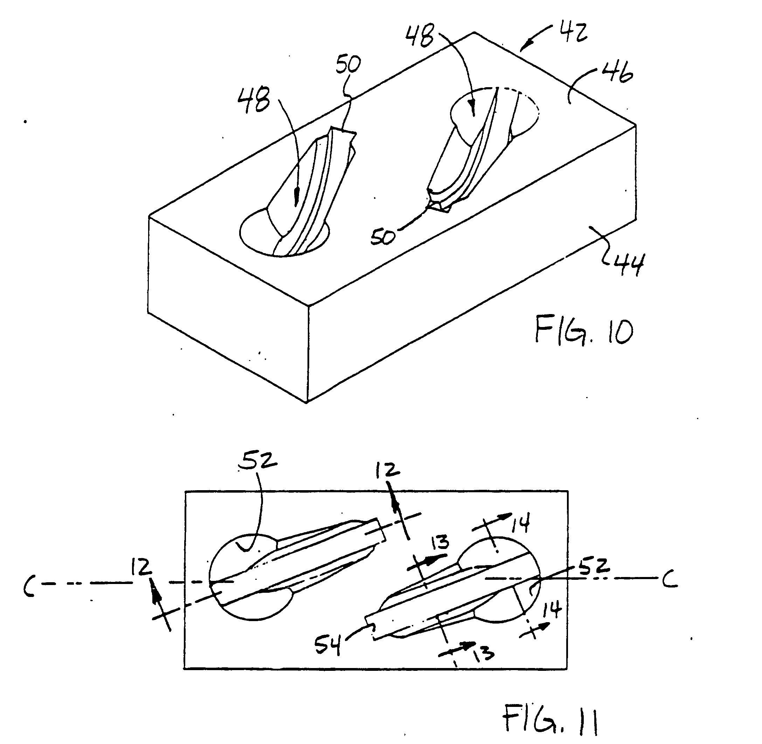

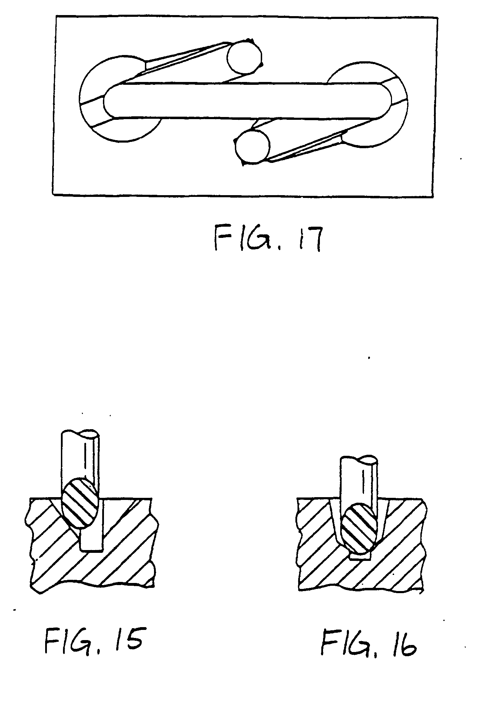

Figures 10 through 17 show a surgical stapling device anvil in accordance with the present invention. This anvil 42 according to the invention differs from that shown in reference Figures 7 through 9 by providing a supporting structure 44 having a staple-receiving face 46 formed with alternatively shaped pocket-like depressions 48. More particularly, the face 46 includes two anvil pockets 48 each having a contoured staple-forming groove 50. The staple-forming grooves 50 extend in a direction parallel to each other but canted relative to the longitudinal axis C-C of the anvil so the staple legs when deformed are again offset to opposite sides of the back span of the staple.

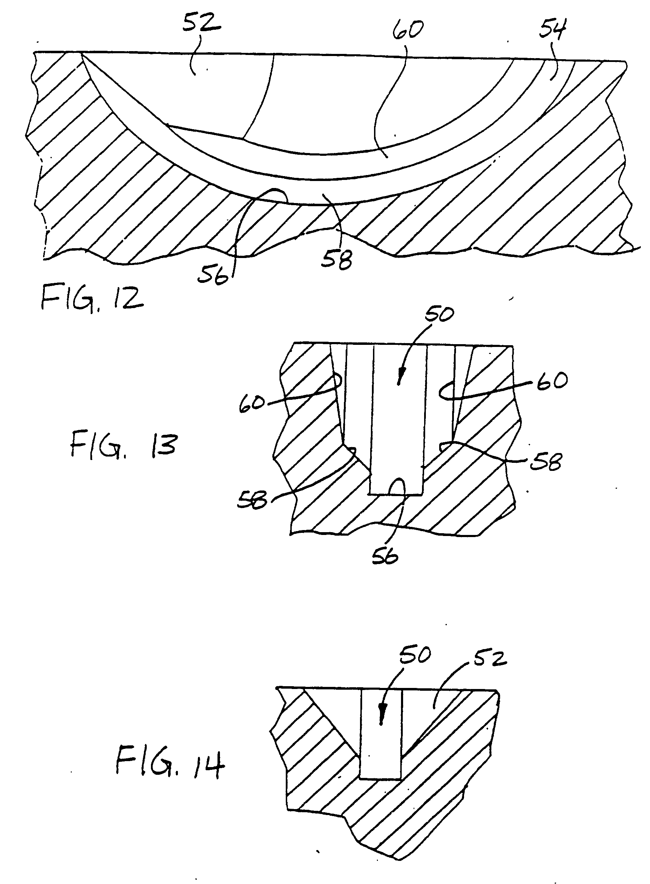

Each pocket has a generally arcuate longitudinal configuration, as shown in Figure 12, that extends from a wide conical entry end 52 to a narrow exit end 54. That is, the conical entry end 52 has a diameter at the face 46 which is larger than the diameter of a staple leg and point as can be seen in Figure 15, whereas the width of the exit end 54 should be smaller than the diameter of a staple leg or point as depicted in Figures 15 and 16. The bottom of the conical entry end 52 leads smoothly to the floor 56 of the pocket, which is defined by the staple guiding groove 50. At its center near its lowest section and extending toward the exit end, the groove 50 in each pocket is bounded on its lateral sides by stepped sloping walls 58 and 60 that narrow the pocket in that region as shown in Figures 11 and 13. As can be seen in Figure 13 the surface 58 has a steeper slope than does the surface 60. Ultimately the groove 50 in each pocket terminates at the face in its exit end 54 which is the narrowest section of the pocket.

As can be seen in Figures 13 and 16, the floor 56 of the groove 50 lies significantly below the lower extreme of the surfaces 58. Thus the apex of the chiseled staple point is prevented from engaging and thereby digging into the floor 56.

Moreover, while the entry end 52 is relatively large, at the surface 46 it quickly necks down to a relatively narrow configuration. This arrangement minimizes "tenting" or pushing of tissue into the anvil pocket by a staple driven into the pocket.

Thus it will be understood that this anvil configuration provides a relatively large target, the conical entry end 52, for each end point 16' of a staple leg 14' at the start of staple driving. The end point can be received in the entry end off center as shown in Figure 15, so certain variations from staple to staple can be tolerated. However, as driving continues the staple end point is quickly guided to the groove 50 by the sloping walls 58 and 60 as shown in Figure 16. Finally the end point is guided to exit from the groove itself at the exit end 54 of the pocket, without digging into the floor of the groove, as driving is completed, thereby to form the staple into the fully deformed configuration, as shown in Figure 17.

Again, the anvil in accordance with this embodiment is arranged in the stapling device so that its longitudinal axis C-C is parallel to the back span of a staple held to be driven theretoward. Driving of the staple by the device toward the anvil may then proceed in the same way as described with reference to the embodiment shown in reference Figures 7 to 9.

As will be readily appreciated by those skilled in the art, the present invention provides marked improvements over known stapling devices. Moreover, by taking advantage of the unique properties of recently developed malleable, bioabsorbable, polymeric staples, this invention provides a unique deformed staple shape.

Although a specific embodiment of the present invention has been described above in detail, it will be understood that this description is merely for purposes of illustration. Various modifications of and equivalent structures corresponding to the disclosed aspects of the preferred embodiment in addition to those described above may be made by those skilled in the art.

## Claims

1.  Stapling device for applying a surgical staple comprising in combination:

    (a) an anvil for said staple, with its first and second legs (14') extending in one direction from opposite ends of the back span (12') and substantially perpendicularly thereto in an undeformed state; said anvil comprising: a supporting body (44) having a longitudinal axis (C-C) and including a bioabsorbable polymeric surgical staple-receiving face (46); first and second pocket depressions (48)each beginning with an entry end (52) located at said staple-receiving face (46), continuing to a depressed portion within said body (44) below said staple-receiving face (46), and terminating in an exit end (54) at said staple-receiving face (46), said first and second pocket depressions (48) extending in non-collinear relation with the entry end (52) of each located substantially on the longitudinal axis and the exit end (54) of each located on a side of the longitudinal axis opposite the side on which the exit end (54) of the other pocket depression is located, said entry ends (52) of said first and second pocket depressions (48) being spaced by a distance substantially equal to the distance between the first and second legs (14') of the staple in the undeformed state; whereby the first and second legs (14') of a staple driven toward said anvil and received in the entry ends (52) of the respective first and second pocket depressions (48) are guided to said exit ends (54)thereof to be deformed toward the back span (12') and to extend toward opposite sides of the back span (12') to suture tissue received therein; and

    (b) one-piece, bioabsorbable, malleable, polymeric surgical staples, loaded in an undeformed state in that stapling device and comprising a back span (12') and first and second legs (14') at opposite ends of the back span whereby for receiving tissue therebetween the first and second legs extend in an initial undeformed configuration from opposite ends of the back span (12') in one direction substantially perpendicular to the longitudinal axis of the back span and whereby each leg terminates in an end point (16'), whereby the first and second legs (14') are capable of being plastically deformed initially inwardly toward each other and then toward said back span (12') in a direction generally opposite said one direction to locate the two end points (16') laterally at opposite sides of said back span (12'), wherein further the first and second legs (14')and the back span (12') each has a noncircular cross-section shape, whereby the first and second pocket depressions (48) each have a contoured staple-guiding groove (50) having a floor (56) and being bounded on its lateral sides at its center near its lowest section and extending toward the exit end by stepped sloping walls that narrow the pocket in that region, whereby the floor (56) of the groove (50) lies below the extreme of the surfaces of the walls such that the apex of the chiseled staple point is prevented from engaging and thereby digging into the floor (56).

2.  The stapling device of claim 1, wherein said first and second pocket depressions each comprise first and second walls sloping inwardly of said supporting body toward each other to form a staple leg guide path (36).

3.  The stapling device according to claim 1, wherein said supporting body and said pocket depressions therein are formed of injection molded plastic.

## Patentansprüche

1.  Klammergerät zum Anwenden einer chirurgischen Klammer in Kombination aufweisend:

    (a) einen Amboss für die Klammer, wobei deren erstes und zweites Bein (14') in einem nicht verformten Zustand in einer Richtung von gegenüberliegenden Enden des hinteren Bügels (12') und im Wesentlichen senkrecht dazu verlaufen; wobei der Amboss umfasst: einen Auflagekörper (44), der eine Längsachse (C-C) aufweist und eine eine bioabsorbierbare polymere chirurgische Klammer aufnehmende Fläche (46) enthält; erste und zweite Taschenvertiefungen (48), die jeweils mit einem Eintrittsende (52) beginnen, das bei der eine Klammer aufnehmenden Fläche (46) liegt, sich zu einem vertieften Abschnitt innerhalb des Körpers (44) unter der eine Klammer aufnehmenden Fläche (46) fortsetzen und in einem Austrittsende (54) an der eine Klammer aufnehmenden Fläche (46) enden, wobei die ersten und zweiten Taschenvertiefungen (48) nicht kollinear verlaufen, wobei das Eintrittsende (52) von jeder im Wesentlichen auf der Längsachse liegt und das Austrittsende (54) von jeder auf einer Seite der Längsachse liegt, die der Seite gegenüberliegt, auf der das Austrittsende (54) der anderen Taschenvertiefung liegt, wobei die Eintrittsenden (52) der ersten und zweiten Taschenvertiefung (48) in einer Distanz beabstandet sind, die im Wesentlichen gleich der Distanz zwischen dem ersten und zweiten Bein (14') der Klammer im nicht verformten Zustand ist; wodurch das erste und zweite Bein (14') einer in Richtung auf den Amboss getriebenen und in den Eintrittsenden (52) der jeweiligen ersten und zweiten Taschenvertiefung (48) aufgenommenen Klammer zu deren Austrittsenden (54) geführt werden, so dass sie in Richtung auf den hinteren Bügel (12) verformt werden und sich zu gegenüberliegenden Seiten des hinteren Bügels (12') erstrecken, um darin aufgenommenes Gewebe zu nähen; und

(b) einteilige bioabsorbierbare verformbare polymere chirurgische Klammern, die in einem nicht verformten Zustand in dem Klammergerät geladen sind und einen hinteren Bügel (12') und erste und zweite Beine (14') an gegenüberliegenden Seiten des hinteren Bügels aufweisen, wodurch, um dazwischen Gewebe aufzunehmen, die ersten und zweite Beine in einer anfänglichen nicht verformten Konfiguration von gegenüberliegenden Enden des hinteren Bügels (12') in einer Richtung verlaufen, die zur Längsachse des hinteren Bügels im Wesentlichen senkrecht ist, und wodurch jedes Bein in einer Endspitze (16') endet, wodurch das erste und zweite Bein (14') anfangs einwärts aufeinander zu und dann in Richtung auf den hinteren Bügel (12') in einer zu der einen Richtung im Wesentlichen entgegengesetzten Richtung plastisch verformt werden können, um die beiden Endspitzen (16') seitlich an gegenüberliegenden Seiten des hinteren Bügels (12') anzuordnen, wobei ferner das erste und zweite Bein (14') und der hintere Bügel (12') jeweils eine nicht kreisförmige Querschnittform aufweisen, wodurch die erste und zweite Taschenvertiefung (48) jeweils eine konturierte Klammern führende Rille (50) aufweisen, die einen Boden (56) hat und auf ihren lateralen Seiten an ihrer Mitte nahe ihrem tiefsten Teil begrenzt ist und in Richtung auf das Austrittsende entlang gestuften schrägen Wänden verläuft, die die Tasche in dieser Zone verengen, wodurch der Boden (56) der Rille (50) unter dem äußersten Ende der Oberflächen der Wände liegt, so dass verhindert wird, dass der Scheitel der zugeschnittenen Klammerspitze in Eingriff kommt und sich dadurch in den Boden (56) gräbt.

**2.** Klammergerät nach Anspruch 1, worin die ersten und zweiten Taschenvertiefungen jeweils erste und zweite Wände aufweisen, die schräge einwärts des Auflagekörpers aufeinander zu verlaufen, um einen Weg (36) zur Führung eines Klammerbeins zu bilden.

**3.** Klammergerät nach Anspruch 1, worin der Auflagekörper und die Taschenvertiefungen darin aus Spritzgusskunststoff geschaffen sind.

## Revendications

**1.** Agrafeuse permettant d'appliquer une agrafe chirurgicale comprenant :

a) une touche fixe pour ladite agrafe avec ses première et deuxième jambe (14') s'étendant dans une direction à partir des extrémités opposées de la portée arrière (12') et étant sensiblement perpendiculaire à cette portée dans un état non déformé, ladite touche fixe comprenant : un support (44) possédant un axe longitudinal (C-C) et comprenant une face de réception d'agrafe chirurgicale polymére bioabsorbable (46), des premiers et des deuxièmes évidements de poche (48) commençant chacun par une extrémité d'entrée (52) située sur ladite face de réception d'agrafe (46) et se prolongeant en une partie abaissée dans ledit corps (44) située sous ladite face de réception d'agrafe (46) et se terminant dans une extrémité de sortie (54) de ladite face de réception d'agrafe (46), lesdits premiers et deuxièmes évidements de poche (48) s'étendant suivants une relation non colinéaire avec l'extrémité d'entrée (52) de chaque élément situé essentiellement sur l'axe longitudinal et avec l'extrémité de sortie (54) de chaque élément situé sur un côté du côté opposé à l'axe longitudinal sur lequel se trouve l'extrémité de sortie (54) de l'autre évidement de poche, lesdites extrémités d'entrée (52) desdits premiers et deuxièmes évidements de poche (48) étant espacées d'une distance sensiblement égale à la distance entre la première et la deuxième jambe (14') de l'agrafe dans un état non déformé ; moyennant quoi les première et deuxième jambe (14') d'une agrafe actionnée vers ladite touche fixe et reçue dans les extrémités d'entrée (52) des premiers et des deuxièmes évidements de poche (48) respectifs sont guidées vers lesdites extrémités de sortie (54) devant être déformées vers la portée arrière (12') et devant s'étendre vers les côtés opposés de la portée arrière (12') afin de suturer le tissu reçu et

b) des agrafes compactes chirurgicales polymère bioabsorbables et souples, chargées dans un état non déformé de cette agrafeuse et comprenant une portée arrière (12') ainsi que la première et la deuxième jambes (14') sur les extrémités opposées de la portée arrière, moyennant quoi, pour recevoir le tissu, la première et la deuxième jambe s'étendent dans une configuration initiale non déformée à partir des extrémités opposées de la portée arrière (12') dans une direction sensiblement perpendiculaire à l'axe longitudinal de la portée arrière et moyennant quoi chaque jambe se termine par un point final (16'), moyennant quoi la première et la deuxième jambes (14') peuvent être déformées de manière plastique initialement en interne l'une vers l'autre, puis vers ladite portée arrière (12') dans une direction généralement opposée à une dite direction afin de localiser les deux points finaux (16') latéralement sur les côtés opposés de ladite portée arrière (12'), la première et la deuxième jambe (14') ainsi que la portée arrière (12') possédant en outre chacune une forme de coupe non circulaire, moyennant quoi le premier et le deuxième évidement de poche (48) possèdent chacun une rainure de guidage d'agrafe (50) à contours possédant un sol (56) et étant reliée sur ses côtés latéraux en son centre à côté de sa section la plus basse et s'étendant vers l'extrémité de sortie par des parois inclinées à étages qui rétrécissent la poche dans cette zone, moyennant quoi le sol (56) de la rainure (50) se trouve sous la partie extrême des surfaces des parois de telle sorte que l'apex du point d'agrafe ciselé ne peut pas s'intégrer et ainsi s'enfoncer dans le sol (56).

2.  Agrafeuse selon la revendication 1 dans laquelle lesdits premiers et des deuxièmes évidements de poche comprennent chacun une première et une deuxième parois inclinées l'une vers l'autre, à l'intérieur dudit support, pour former un chemin de guidage (36) pour les jambes de l'agrafe.

3.  Agrafeuse selon la revendication 1, dans laquelle ledit support et lesdits évidements de poche sont composés de matière plastique fondue par injection.

FIG. 1
(PRIOR ART)

FIG. 2
(PRIOR ART)

FIG. 3 (PRIOR ART)

FIG. 4

FIG. 5

FIG. 6

FIG. 6A

FIG. 6B

FIG. 7
(REFERENCE)

FIG. 8
(REFERENCE)

FIG. 9
(REFERENCE)

FIG. 10

FIG. 11

FIG. 12

FIG. 13

FIG. 14

FIG. 17

FIG. 15

FIG. 16